# EUROPÄISCHE PATENTANMELDUNG

(11) **EP 4 714 534 A2**
(43) Veröffentlichungstag der Anmeldung: **25.03.2026**
(21) Anmeldenummer: 26157493.3
(22) Anmeldetag: 03.03.2023
(51) Int. Cl.: B01F 31/22, B01F 31/20, B01F 35/42, C12M 3/06

(54) **VORRICHTUNG ZUM SCHÜTTELN VON PROBEN**

(30) Priorität: 04.03.2022 WO PCT/EP2022/055636
(62) Teilanmeldung aus: 23709655.7
(71) Anmelder: Infors AG, 4103 Bottmingen (CH)
(72) Erfinder: Hawrylenko, Alexander, 4103 Bottmingen (CH); Doppler, Johan, 68480 Lutter (FR)
(74) Vertreter: E. Blum & Co. AG

(57) **Zusammenfassung**

Die Erfindung betrifft eine Vorrichtung zum Schütteln von Proben umfassend einen Träger (62), ein Antriebselement (66), das drehbar am Träger (62) gelagert ist, und ein Tablar (61) eingerichtet zur Beladung mit den Proben (69). Weiterhin umfasst die Vorrichtung eine Tablarwelle (67), die mit dem Tablar (61) verbunden und exzentrisch an dem Antriebselement (66) gelagert ist, sowie ein öffenbares Gehäuse (64). Das Tablar (61), die Tablarwelle (67), das Antriebselement (66) und der Träger (62) befinden sich in einem Innenraum innerhalb des Gehäuses (64). Ausserdem umfasst die Vorrichtung eine durch einen Antrieb (65) antreibbare Hauptantriebswelle (63), die orthogonal zu dem Tablar (61) zumindest teilweise durch das Gehäuse (64) verläuft. Das Antriebselement (66) ist über die Hauptantriebswelle (63) antreibbar. Der Träger (62) samt Tablar (61) ist um die Hauptantriebswelle (63) schwenkbar gelagert, sodass der Träger (62) samt Tablar (61) zumindest teilweise aus dem Gehäuse (64) im geöffneten Zustand heraus schwenkbar ist.

## Beschreibung

### Gebiet der Erfindung

Die Erfindung betrifft eine Vorrichtung zum Schütteln von Proben, insbesondere einen Laborschüttler, insbesondere für das Schütteln und/oder Durchmischen von Flüssigkeit enthaltenden Proben.

### Hintergrund

Schüttler werden verwendet, um Flüssigkeiten, z.B. Zellkulturen, Biotreibstoffe oder Blutproben, in Gefässen zu schütteln und/oder zu durchmischen. Häufig umfasst die geschüttelte Einheit ein Tablar, auf welchem sich die Gefässe, z.B. Erlenmeyerkolben, Reagenzgläser oder sonstige Ampullen, mit den Proben befinden. Dabei ist einerseits für eine gute Durchmischung eine hohe Schüttelfrequenz wünschenswert. Andererseits kann beim Schüttelvorgang Flüssigkeit verschüttet oder verspritzt werden, wobei auch weitere Bauteile wie ein Gehäuse um das Tablar und/oder ein Antrieb des Schüttlers kontaminiert werden können. Bei herkömmlichen Schüttlern sind die Tablare nur mit erheblichem Aufwand entfernbar. Somit stellt auch die Reinigung nach einer Kontamination einen erheblichen Aufwand dar, besonders da einige Bauteile aufgrund des Gehäuses schwer zugänglich sind. Die schlechte Zugänglichkeit der Tablare und Proben erschwert bei herkömmlichen Schüttlern auch die Automatisierung, z.B. die Manipulation der Proben über einen Roboterarm.

Es stellt sich daher die Aufgabe, eine Vorrichtung zum Schütteln von Proben bereitzustellen, die eine leichte Bedienbarkeit aufweist, wobei die Tablare gut zugänglich sind und dadurch insbesondere der Reinigungsaufwand bei einer Kontamination möglichst gering und/oder eine automatisierte Manipulation der Proben möglich ist.

### Darstellung der Erfindung

Diese Aufgabe wird gelöst von einer Vorrichtung zum Schütteln von Proben gemäss Anspruch 1. Unter einer Probe wird dabei insbesondere ein Stoff oder eine Stoffzusammensetzung verstanden, welche eine Flüssigkeit enthält, z.B. eine Zellkultur, ein Biotreibstoff oder eine Blutprobe. Die Probe ist üblicherweise in einem Gefäss, z.B. einem Reagenzglas oder einer Mikrotiterplatte, enthalten, das wiederum von einem Gefässhalter oder -ständer gehalten werden kann.

Die Vorrichtung umfasst
- einen Träger: Der Träger ist insbesondere eingerichtet zum Tragen oder Unterstützen eines Tablars;
- ein Antriebselement, das drehbar am Träger gelagert ist: Das Antriebselement kann beispielsweise eine Hohlwelle oder eine Antriebsscheibe umfassen;
- ein Tablar eingerichtet zur Beladung mit den Proben: Insbesondere ist das Tablar ein flächiges Element, auf dem die Proben angebracht werden können. Vorteilhafterweise umfasst das Tablar Befestigungselemente zum Befestigen mindestens eines Gefässes mit einer Probe bzw. zum Befestigen mindestens eines Gefässhalters oder -ständers;
- eine Tablarwelle, die, insbesondere fest, mit dem Tablar verbunden und exzentrisch an dem Antriebselement gelagert ist: Vorteilhafterweise ist die Tablarwelle zentral an dem Tablar befestigt, insbesondere nahe eines Schwerpunkts des Tablars bzw. des Tablars mit einer vorgesehenen Beladung mit Proben. Als "nahe" ist insbesondere ein Bereich um den Schwerpunkt bis zu +/-10% von Länge und Breite des Tablars bezeichnet. Durch die zentrale Befestigung unterstützt die Tablarwelle (bei vorgesehener Beladung) das Tablar nahe dem Schwerpunkt;

Als Exzentrizität (der Lagerung) der Tablarwelle an dem Antriebselement wird der Abstand zwischen einer Drehachse der Tablarwelle und einer Drehachse des Antriebselements bezeichnet, welche insbesondere parallel zueinander verlaufen. Die Exzentrizität bestimmt eine Auslenkung des Tablars (und damit der Proben) beim Schütteln. Insbesondere kann die Exzentrizität der Lagerung der Tablarwelle an dem Antriebselement zwischen 0.5 und 50 mm betragen, insbesondere zwischen 1 und 3 mm. Dies führt zu einer Auslenkung des Tablars, welche für das Schütteln von Proben in kleineren Gefässen, z.B. Reagenzgläsern oder Mikrotiterplatten, angepasst ist;
- ein öffenbares Gehäuse, wobei sich das Tablar, die Tablarwelle, das Antriebselement und der Träger in einem Innenraum innerhalb des Gehäuses befinden: Das Gehäuse kann einerseits dem Abschluss von Tablar und Proben von einer Umgebung der Vorrichtung dienen. Andererseits kann die Vorrichtung ein Klimasteuerelement umfassen, das eingerichtet ist zum Steuern von Temperatur und/oder Feuchtigkeit in einem Innenraum des Gehäuses. Zu diesem Zweck und insbesondere um ideale Umgebungsbedingungen für die Proben zu schaffen, kann das Klimasteuerelement z.B. einen Heizer, einen Kühler, einen Befeuchter und/oder einen Entfeuchter umfassen, der an dem Gehäuse angebracht sein kann. Zum öffenbaren Verschliessen des Gehäuses und damit zum Aufrechterhalten der idealen Umgebungsbedingungen umfasst das Gehäuse vorteilhafterweise eine Tür, z.B. eine schwenkbare Tür, insbesondere an einer Vorderseite des Gehäuses;
- eine durch einen Antrieb, z.B. umfassend einen Motor, antreibbare Hauptantriebswelle, die orthogonal zu dem Tablar zumindest teilweise durch das Gehäuse verläuft: Vorteilhafterweise verläuft die Hauptantriebswelle vertikal durch das Gehäuse, also insbesondere orthogonal zu einer Auflagefläche des Gehäuses, die z.B. durch eine Ebene durch Füsse am Gehäuse gegeben sein kann. Das Tablar verläuft dann horizontal im Gehäuse, d.h. im bestimmungsgemässen Gebrauch insbesondere orthogonal zur Schwerkraft. Es ist dabei möglich, aber nicht nötig, dass die Hauptantriebswelle durch das Tablar bzw. durch eine Ebene in Verlängerung des Tablars hindurch verläuft.

Das Antriebselement ist über die Hauptantriebswelle antreibbar. Somit kann ein Schütteln des Tablars durch einen Antrieb, z.B. mit einem Motor, über die Hauptantriebswelle und das Antriebselement erreicht werden.

Ausserdem ist der Träger samt Tablar um die Hauptantriebswelle schwenkbar gelagert ist. Die Lagerung des Trägers kann über ein Lager, insbesondere an der Hauptantriebswelle selbst oder am Gehäuse bzw. einem am Gehäuse befestigten Trägerelement, ausgeführt sein. Durch die schwenkbare Lagerung wird eine bessere Zugänglichkeit des Tablars erzielt, z.B. für ein Be- und Entladen des Tablars mit Proben. Insbesondere wird damit eine Automatisierung der Bedienung des Schüttlers ermöglicht, z.B. über einen computergesteuerten Roboterarm. Gleichzeitig wird damit eine bessere Zugänglichkeit des Innenraums des Gehäuses erreicht, indem das Tablar beispielsweise aus dem Gehäuse heraus geschwenkt wird. Dies wiederum ermöglicht ein einfacheres Reinigen des Innenraums und der darin befindlichen Bauteile und somit ein Arbeiten unter kontrollierten, insbesondere sterilen, Bedingungen.

### Schwenkbares Tablar

Der Träger samt Tablar ist zumindest teilweise, insbesondere zu mehr als 50% oder mehr als 70% einer Fläche des Tablars, aus dem Gehäuse im geöffneten Zustand heraus schwenkbar. In einer Ausführungsform ist der Träger samt Tablar um mindestens 45°, insbesondere mindestens 90°, um die Hauptantriebswelle schwenkbar. Durch diese Massnahmen wird wiederum eine Zugänglichkeit von Tablar und Innenraum des Gehäuses verbessert.

Vorteilhafterweise umfasst das Tablar eine Öffnung, durch welche die Hauptantriebswelle hindurchtritt. Dadurch ist eine kompakte Bauweise der Vorrichtung, insbesondere bei mehreren über die Hauptantriebswelle angetriebenen Tablaren, möglich. Weiterhin ist es vorteilhaft, dass sich die Öffnung in einem Randbereich des Tablars befindet, insbesondere weniger als 20% einer Länge und/oder Breite des Tablars von einem Rand des Tablars entfernt. Durch eine solche Lage der Öffnung bzw. der Hauptantriebswelle relativ zum Tablar lässt sich das Tablar weit aus dem Gehäuse heraus schwenken, also insbesondere zu mehr als 50% oder mehr als 70% der Fläche des Tablars. Besonders geeignet ist hierfür, dass sich die Öffnung in einem Eckbereich des Tablars befindet, insbesondere weniger als 20% der Länge und der Breite des Tablars vom Rand des Tablars entfernt. Bevorzugt befindet sich die Öffnung bzw. die Hauptantriebswelle im Tür-nahen Eckbereich des Tablars.

Alternativ kann die Hauptantriebswelle auch ausserhalb der Fläche des Tablars verlaufen. In diesem Fall kann eine kompakte Bauweise des Gehäuses erreicht werden, indem die Hauptantriebswelle nahe des Tablars verläuft, insbesondere weniger als 20% der Länge oder Breite des Tablars vom Rand des Tablars entfernt. Bevorzugt verläuft die Hauptantriebswelle nahe einer Tür-nahen Ecke des Tablars.

In einer Ausführungsform weist das Tablar eine rechteckige Form auf. Unter "rechteckig" ist dabei jede im Wesentlichen rechteckige Form zu verstehen, z.B. mit abgerundeten Ecken oder ein Parallelogramm oder Trapez. Insbesondere kann das Tablar eine Länge zwischen 50 und 100 cm und/oder eine Breite zwischen 30 und 70 cm aufweisen, was die Beladung mit einer Vielzahl von Proben sowie eine Verwendung von üblichen Gefässen und Gefässhaltern ermöglicht.

Weiterhin umfasst der Träger am Hauptantriebswellen-fernen Ende eine Verrastung zum lösbaren Befestigen des Trägers an dem Gehäuse oder an einer Stützstruktur im Gehäuse. Dies ermöglicht ein besseres Befestigen des Trägers, der das Tablar unterstützt, im Betrieb. Insbesondere ist die Verrastung dazu eingerichtet, den Träger (und damit auch das Tablar) daran zu hindern, um die Hauptantriebwelle zu schwenken. Gleichzeitig ermöglicht die Verrastung, welche z.B. einen Steckverschluss oder eine einrastbare Klappe umfassen kann, ein schnelles und bedienerfreundliches Entriegeln und Herausschwenken des Tablars aus dem Gehäuse.

Ausserdem kann die Vorrichtung einen Riemen umfassen, der eingerichtet ist zum Antreiben des Antriebselements über die Hauptantriebswelle. Insbesondere ist in diesem Fall an dem Antriebselement eine erste Riemenscheibe und an der Hauptantriebswelle eine zweite Riemenscheibe angebracht, über welche der Riemen verläuft. Alternativ ist auch ein Zahnradgetriebe zum Antreiben des Antriebselements über die Hauptantriebswelle denkbar.

Vorteilhafterweise ist das Tablar gegen Rotation gegenüber dem Träger gesichert, insbesondere um bei einer Rotation des Antriebselements nicht oder zumindest nicht wesentlich, insbesondere nicht über 10°, mitzurotieren. Für eine solche Sicherung des Tablars gegen Rotation sind mehrere Varianten denkbar: Einerseits eine mechanische Führung, die am Gehäuse angebracht ist und eingerichtet ist, die Freiheitsgrade des Tablars auf Translationen in der Tablarebene einzuschränken, z.B. über elastische Elemente wie Federn, die insbesondere im Randbereich des Tablars angreifen. Andererseits kann der Antrieb selbst, z.B. über den Riemen, zu einer solchen Sicherung des Tablars gegen Rotation ausgestaltet sein. Hierzu kann die Vorrichtung eine zweite Tablarwelle umfassen, die mit dem Tablar verbunden und exzentrisch, insbesondere mit der gleichen Exzentrizität wie die Tablarwelle, an der Hauptantriebswelle, z.B. an der zweiten Riemenscheibe, gelagert ist. Durch die zweifache Lagerung des Tablars wird eine Rotation ebenfalls verhindert.

In einer besonders vorteilhaften Ausführungsform mit erster und zweiter Riemenscheibe, erster und zweiter Tablarwelle und erstem und zweitem Antriebselement umfasst das Tablar einen ersten Tablarteil und einen zweiten Tablarteil. Die erste und zweite Tablarwelle sind, insbesondere fest, mit dem ersten bzw. zweiten Tablarteil verbunden und exzentrisch an dem ersten bzw. zweiten Antriebselement gelagert. Zur Verhinderung einer Rotation des Tablars sind der erste und der zweite Tablarteil durch eine Linearführung miteinander verbunden. Eine solche Linearführung ist insbesondere dazu eingerichtet, dass sich der erste und der zweite Tablarteil zwar entlang einer Achse der Linearführung gegeneinander verschieben, aber nicht gegeneinander verdrehen können. In anderen Worten beschränkt die Linearführung alle Freiheitsgrade der Tablarteile zueinander mit Ausnahme eines Translationsfreiheitsgrads entlang der Achse der Linearführung. Die Ausführungsform mit zwei Tablarteilen, die mit einer Linearführung verbunden sind, hat den Vorteil, dass schädliche Kräfte auf die Lagerung der Tablarwellen, die z.B. durch eine Wärmeausdehnung eines einstückigen Tablars bewirkt werden, verhindert werden. Damit lassen sich wiederum höhere Schüttelfrequenzen, eine längere Betriebsdauer sowie ein ruhigerer Lauf des Schüttlers erzielen.

In einer weiteren vorteilhaften Ausführungsform umfasst die Vorrichtung weiterhin ein am Gehäuse befestigtes Trägerelement. Der Träger samt Tablar ist über ein Lager, insbesondere über ein Gleitlager, schwenkbar an dem Trägerelement gelagert ist. Dabei fällt vorteilhafterweise eine Schwenkachse des Trägers mit der Hauptantriebswelle zusammen. Insbesondere steht der Träger also nicht in mechanischem Kontakt mit der Hauptantriebswelle. Diese mechanische Trennung von Hauptantriebswelle und Lagerung des Trägers vermindert unerwünschte Reibung, Verschleiss und Vibrationen, gerade bei hohen Schüttelfrequenzen (und damit hohen Rotationsfrequenzen der Hauptantriebswelle).

### Mehrere Tablare

In einer Ausführungsform umfasst die Vorrichtung mindestens ein weiteres Tablar samt weiterer Tablarwelle, weiterem Antriebselement und weiterem Träger im Innenraum des Gehäuses. Insbesondere kann die Vorrichtung mindestens fünf weitere Tablare, samt weiteren Tablarwellen, weiteren Antriebselementen und weiteren Trägern im Innenraum des Gehäuses umfassen. Dadurch wird die Kapazität der Vorrichtung, also insbesondere eine Anzahl gleichzeitig schüttelbarer Proben, erhöht. Vorteilhafterweise ist das mindestens eine weitere Antriebselement bzw. sind die mindestestens fünf weiteren Antriebselemeente ebenfalls über die Hauptantriebswelle antreibbar. So wird eine kompakte Bauweise mit einfacher Wartbarkeit erreicht.

Vorteilhafterweise ist das mindestens eine weitere Tablar unabhängig von dem anderen Tablar bzw. den anderen Tablaren um die Hauptantriebswelle schwenkbar gelagert. Insbesondere können alle Tablare unabhängig voneinander um die Hauptantriebswelle, z.B. aus dem Gehäuse heraus, geschwenkt werden. Dies verbessert wiederum die Zugänglichkeit der Tablare, z.B. beim Be- und Entladen mit Proben.

Um eine Unwucht im Falle mehrerer Tablare zu vermeiden, ist es von Vorteil, dass eine Winkelposition einer Lagerung der weiteren Tablarwelle an dem weiteren Antriebselement abweicht von einer Winkelposition der Lagerung der Tablarwelle an dem Antriebselement. Ansonsten könnte, besonders bei schwerer Beladung der Tablare, eine Unwucht auf die Hauptantriebswelle wirken und die gesamte Vorrichtung zu Vibrationen anregen.

Dies kann insbesondere vermieden werden, indem sich die Winkelposition der Lagerungen der verschiedenen Tablarwellen zueinander um 360°/N unterscheiden, wobei N die Anzahl der Tablare in der Vorrichtung ist. Dadurch wird eine Unwucht auf die Hauptantriebswelle ausgeglichen.

### Antrieb

In einer Ausführungsform umfasst der Antrieb einen Motor, z.B. einen Elektromotor, der ausserhalb des Gehäuses angebracht ist. Der Motor ist insbesondere über ein Getriebe an die Hauptantriebswelle gekoppelt. Die Anbringung des Motors ausserhalb des Gehäuses hat den Vorteil, dass Wärme, welche beim Betrieb des Motors entsteht, nicht in den Innenraum des Gehäuses eingebracht wird. Für viele Anwendungen bzw. Proben ist eine Steuerung von Temperatur und/oder Feuchte, z.B. eine Klimasteuerung wie oben beschrieben, wünschenswert. Dabei wird insbesondere die Feuchtigkeit nahe des Taupunkts gehalten, insbesondere bei einer relativen Feuchte zwischen 80% und 100%. Wenn der Innenraum nun aber gleichzeitig gekühlt werden muss, z.B. wegen des Eintrags von Wärme durch einen Motor im Innenraum, überschreitet die Feuchte lokal an der Klimasteuerung bzw. am Kühler 100% und kondensiert aus. Ein Kondensieren ist wiederum unerwünscht, weil dadurch eine unkontrollierte Vermehrung von Fremdkeime auftreten kann, was schädlich für die Proben sein kann. Insbesondere soll der Innenraum des Gehäuses thermisch von dem Motor entkoppelt sein. Dieses Problem wird durch einen ausserhalb des Gehäuses angebrachten Motor gelöst.

Insbesondere kann der Motor an einer Unterseite des Gehäuses angebracht sein. Dies setzt einen Schwerpunkt der Vorrichtung herunter, also näher zur Auflagefläche, und erhöht somit die Standfestigkeit der Vorrichtung, insbesondere bei hohen Schüttelfrequenzen. Dabei ist die Hauptantriebswelle vorteilhafterweise durch eine Öffnung in der Unterseite des Gehäuses hindurchgeführt.

Im Allgemeinen ist es vorteilhaft, dass die Schüttelfrequenz des Tablars infolge des Antriebs mindestens 1000 rpm, insbesondere mindestens 1500 rpm, mindestens 2000 rpm oder mindestens 2500 rpm, z.B. bei 3 mm Durchmesser der Kreisbewegung, beträgt. Eine solch hohe Schüttelfrequenz ist besonders zum Schütteln und Durchmischen von Proben in kleinen Gefässen, z.B. in Reagenzgläsern oder in Mikrotiterplatten, geeignet. Ähnliche Kräfte auf die Proben werden z.B. bei Schüttelfrequenz von 350 rpm und einem Durchmesser der Kreisbewegung von 50 mm erzeugt. Insbesondere führen hohe Schüttelfrequenzen von mindestens 1000 rpm, mindestens 1500 rpm, mindestens 2000 rpm oder mindestens 2500 rpm zu einer besseren Durchmischung der geschüttelten Proben sowie zu einem schnelleren Sauerstoff-Transfer von der Gasphase in die Flüssigphase, wodurch ein gutes Wachstum von Zellkulturen in den Proben ermöglicht wird.

### Innenraum des Gehäuses

Um ein Reinigen des Innenraums weiter zu vereinfachen und eine kontrollierte Umgebung für die Proben zu schaffen, kann die Vorrichtung gemäss den Vorgaben für "Hygienic Design" ausgestaltet sein, wie sie z.B. in der Norm ISO 14159 "Safety of machinery - Hygiene requirements for the design of machinery" oder in diversen Artikeln der FDA CFR 177 vorgegeben sind.

Insbesondere kann die Vorrichtung folgende vorteilhafte Merkmale aufweisen:
- Ein dem Innenraum zugewandter Teil des Gehäuses kann zumindest zu 50%, insbesondere zumindest zu 75%, mit rostfreiem Stahl bedeckt sein.
- Der dem Innenraum zugewandte Teil des Gehäuses kann abgerundete Ecken und Kanten umfassen. Vorteilhafterweise beträgt ein Radius der Ecken und Kanten mindestens 10 mm, insbesondere mindestens 15 mm.
- Zumindest ein Teil, insbesondere mindestens 70%, einer Unterseite des Innenraums kann gegenüber der Auflagefläche des Gehäuses schräg verlaufen. Dadurch sammelt sich verspritztes oder übergelaufenes Probenmaterial im tiefsten, insbesondere Auflageflächen-nächsten, Bereich der Unterseite und kann dort einfach entfernt werden. Vorteilhafterweise beträgt ein Winkel zwischen der Unterseite des Innenraums und der Auflagefläche des Gehäuses dabei zwischen 1° und 30°, insbesondere zwischen 5° und 15°.
- Zusätzlich ist es vorteilhaft, dass das Gehäuse im Auflagefläche-nächsten Bereich der Unterseite des Innenraums eine Auslassöffnung, insbesondere durch das Gehäuse hindurch, umfasst. Auch im Hinblick auf eine mögliche Klimasteuerung kann die Auslassöffnung verschliessbar sein.

Jedes dieser Merkmale verbessert alleine oder in Kombination mit den anderen Merkmalen die Reinigbarkeit des Innenraums und der darin befindlichen Bauteile.

Die beschriebenen Ausführungsformen und Merkmale sollen, soweit sinnvoll realisierbar, in beliebiger Kombination als offenbart gelten. Gerade im Hinblick auf eine einfache Handhabbarkeit und gute Reinigbarkeit weisen sie synergistische Wirkungen und Vorteile auf.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1a eine perspektivische Ansicht einer Vorrichtung zum Schütteln von Proben gemäss einer Ausführungsform der Erfindung; Fig. 1b eine Draufsicht von oben auf die Vorrichtung von Fig. 1a; Fig. 1c eine perspektivische Ansicht einer Vorrichtung zum Schütteln von Proben gemäss einer weiteren Ausführungsform der Erfindung;
Fig. 2 einen schematischen Schnitt durch eine Vorrichtung zum Schütteln von Proben mit innenliegendem Motor gemäss dem Stand der Technik;
Figs. 3a und 3b schematische Schnitte durch jeweils eine Vorrichtung zum Schütteln von Proben mit aussenliegendem Motor gemäss Ausführungsformen der Erfindung;
Fig. 4a eine Draufsicht von oben auf eine Ausführungsform der erfindungsgemässen Vorrichtung; Fig. 4b einen schematischen Schnitt entlang der Linie AA' in Fig. 4a; Fig. 4c eine Detailansicht des Eckbereichs B von Fig. 4b;
Fig. 5 eine schematische Seitenansicht einer Ausführungsform der erfindungsgemässen Vorrichtung mit mehreren Tablaren;
Fig. 6a einen schematischen Schnitt vertikal durch eine Vorrichtung zum Schütteln von Proben gemäss einer Ausführungsform; Fig. 6b eine Detailansicht des Bereichs C von Fig. 6a; Fig. 6c einen schematischen Schnitt vertikal durch eine Vorrichtung zum Schütteln von Proben gemäss einer weiteren Ausführungsform;
Fig. 7 einen horizontalen Schnitt bzw. eine Draufsicht von oben auf ein Tablar und ein Gegengewicht gemäss einer Ausführungsform der Erfindung;
Figs. 8a, 8b und 8c Schemazeichnungen einer flüssigen Probe in einem Gefäss bei anwachsender Schüttelfrequenzen;
Fig. 9 einen schematischen Schnitt durch ein Lager, mit dem das Tablar gemäss einer Ausführungsform an der Hauptantriebswelle gelagert ist;
Fig. 10a eine perspektivische Ansicht eines schwenkbaren Tablars mit einer Verrastung, welche in Fig. 10b vergrössert dargestellt ist, gemäss einer Ausführungsform;
Figs. 11a, 11b und 11c eine Ausführungsform mit geteiltem Tablar in einer perspektivischen Ansicht (Fig. 11a), einem schematischen horizontalen Schnitt (Fig. 11b) und einem schematischen vertikalen Schnitt (Fig. 11c).

### Wege zur Ausführung der Erfindung

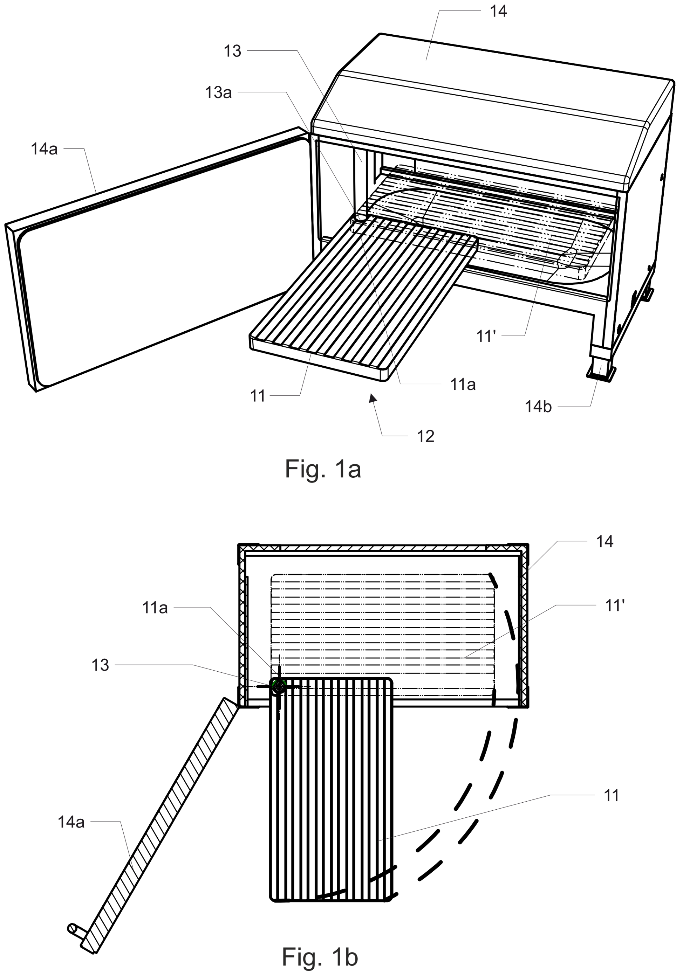

Figs. 1a und 1b zeigen eine Vorrichtung zum Schütteln von Proben, einen sogenannten Schüttler, gemäss einer Ausführungsform. Dabei ist Fig. 1a eine perspektivische Ansicht der Vorrichtung, während Fig. 1b eine Draufsicht von oben wiedergibt. Die Vorrichtung umfasst ein Tablar 11, das mit Proben beladen werden kann. Das Tablar 11 ist auf einem Träger 12 (in Figs. 1a und 1b nicht sichtbar) gelagert und wird von diesem gestützt. Der Träger 12 und mit ihm das Tablar 11 sind um eine Hauptantriebswelle 13 schwenkbar. Zu diesem Zweck ist der Träger 12 über ein Lager 13a mit der Hauptantriebswelle verbunden. Ausserdem weist das Tablar 11 an seinem Randbereich eine Öffnung 11a auf, durch welche die Hauptantriebswelle 13 hindurchtritt. Die Öffnung 11a ist dabei abhängig von der Exzentrizität der Lagerung des Tablars, insbesondere von der Auslenkung der Schüttelbewegung, grösser als ein Durchmesser der Hauptantriebswelle 13.

Das Tablar 11 umfasst mit Vorteil zumindest an seiner Oberseite, also der den Proben zugewandten Seite, eine leicht zu reinigende Oberfläche, z.B. aus Metall. Dies ermöglicht einen sterilen Betrieb der Vorrichtung. Weiterhin kann das Tablar 11 eine Standardgrösse von 850 mm x 470 mm aufweisen.

Das Tablar 11 im eingeklappten Zustand 11' sowie zumindest ein Teil der Hauptantriebswelle 13 sind von einem Gehäuse 14 umschlossen, das zum Öffnen und Verschliessen eine Tür 14a umfasst. Das Gehäuse 14 erfüllt im Allgemeinen mehrere Funktionen: Zum einen bildet es einen ortsfesten Rahmen, der, z.B. über Füsse 14b, auf einem Tisch, in einem Labor oder allgemein auf einer Unterlage platziert werden kann. Die Schüttelbewegung des Tablars geschieht relativ zu diesem ortsfesten Rahmen. Zum zweiten bietet das Gehäuse einen Schutz der Umgebung der Vorrichtung, z.B. vor Verspritzen oder Überlaufen von Proben oder vor Dämpfen, was insbesondere bei schädlichen Proben oder in einem sterilen Labor wünschenswert ist. Zum dritten können in einem Innenraum des Gehäuses kontrollierte Bedingungen, z.B. in Bezug auf Temperatur und/oder Feuchtigkeit, eingestellt werden, wie es für viele Proben vorteilhaft ist. Zu diesem Zweck kann die Vorrichtung eine Klimasteuerung für den Innenraum umfassen (in Figs. 1a und 1b nicht gezeigt).

In Figs. 1a und 1b ist das Tablar in zwei Positionen gezeigt: einerseits (mit 11 bezeichnet) aus dem Gehäuse 14 herausgeschwenkt, andererseits (gestrichelt, mit 11' bezeichnet) im Gehäuse eingeschwenkt in betriebsbereitem Zustand. In der Figur beträgt der Winkel zwischen den beiden Positionen 90°. Im Allgemeinen ist aber bereits ein Winkel von mindestens 45° vorteilhaft, da er die Zugänglichkeit des Tablars 11 und des Innenraums des Gehäuses 14 verbessert.

Weiterhin ist in Figs. 1a und 1b erkennbar, dass eine Anbringung der Hauptantriebswelle 13 im Randbereich oder gar Eckbereichs des Tablars (wie oben definiert) vorteilhaft ist für die Schwenkbarkeit und Zugänglichkeit des Tablars. Alternativ kann der gleiche Vorteil dadurch erreicht werden, dass die Hauptantriebswelle 13 ausserhalb des Tablars 11 nahe dessen Randbereich verläuft (nicht gezeigt in Figs. 1a und 1b). In diesem Fall ist die Öffnung 11a im Tablar 11 überflüssig und der Träger 12 ragt in horizontaler Richtung über das Tablar 11 hinaus. Im Allgemeinen verbessert die Schwenkbarkeit des Tablars aus dem Gehäuse heraus die Zugänglichkeit der Proben. Insbesondere ermöglicht ein schwenkbares Tablar eine Automatisierung des Befüll- und Entnahmeprozesses der Proben, da z.B. ein Roboterarm die Vorrichtung einfacher computergesteuert bedienen kann.

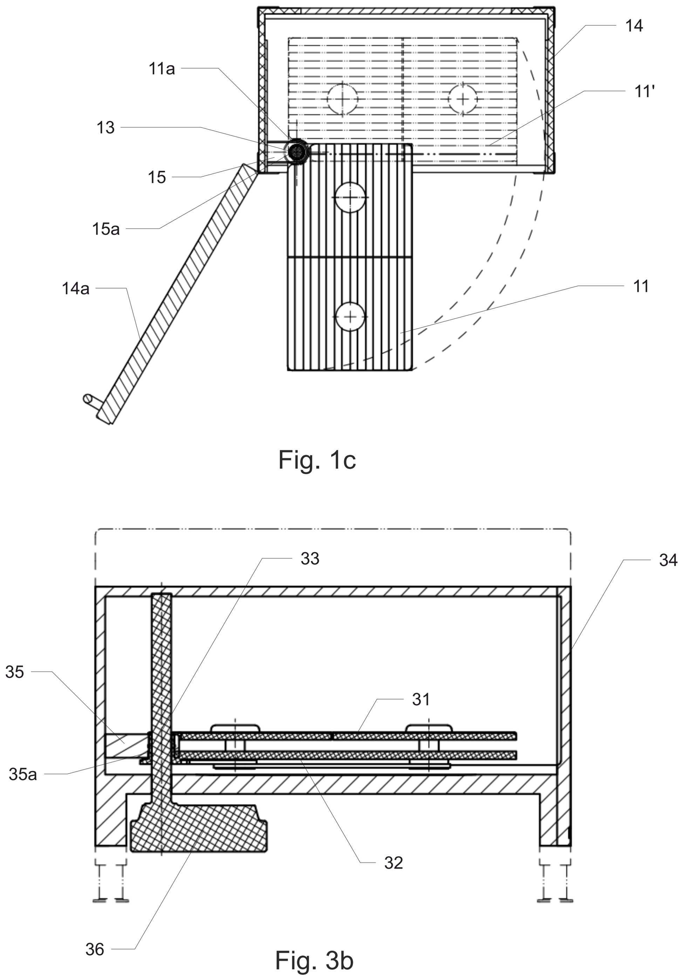

Die genannten Vorteile werden auch durch die modifizierte Lagerung des Trägers 12 samt Tablar 11 wie in Fig. 1c erreicht. Im Unterschied zur Anordnung von Fig. 1a ist der Träger 12 hier über ein Lager 15a, insbesondere ein Gleitlager, an einem Trägerelement 15 gelagert, das ein Teil des Gehäuses 14 sein oder am Gehäuse 14 befestigt sein kann. Dadurch wird der Träger 12 mechanisch von der Hauptantriebswelle 13 entkoppelt. Dies führt zu einer erhöhten Laufruhe und weniger Verschliess an der Vorrichtung.

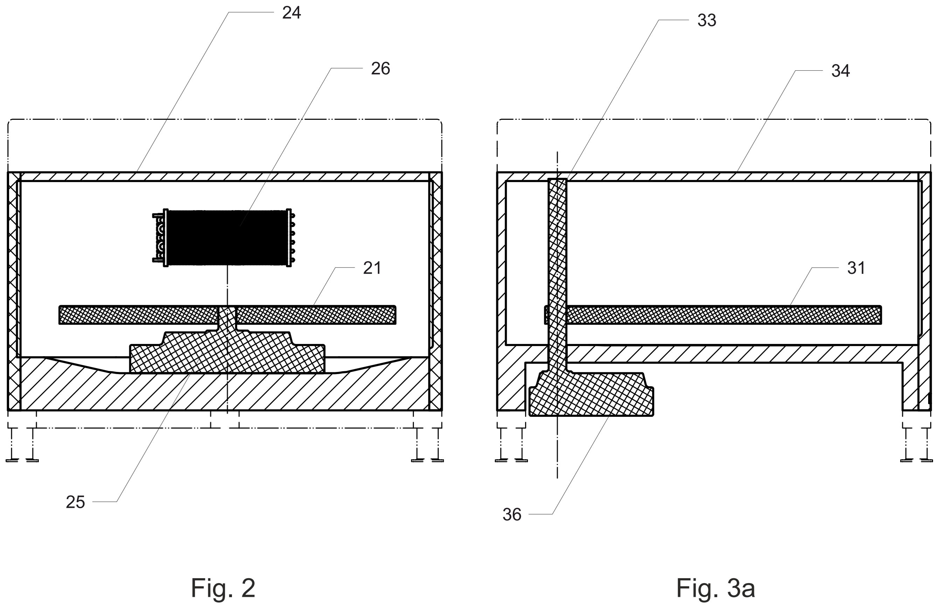

Fig. 2 zeigt einen schematischen Schnitt durch einen Schüttler gemäss dem Stand der Technik. Ein Tablar 21 wird von einem Motor 25 angetrieben, welcher sich innerhalb eines Gehäuses 24 befindet. Die Anordnung des Motors 25 im Innenraum des Gehäuses 24 hat den Nachteil, dass Wärme, die vom Motor 25 generiert wird, direkt den Innenraum und damit darin befindliche Proben erwärmt. Für einige Proben ist es allerdings erforderlich, den Innenraum zu klimatisieren, insbesondere Temperatur und/oder Feuchtigkeit zu steuern, z.B. über eine Klimasteuerung 26. Wie oben beschrieben, kann dies zum Kondensieren von Feuchtigkeit im Innenraum, insbesondere an der Klimasteuerung 26 bzw. dem Kühler, führen, was wiederum die Proben beschädigen kann. Ein innenliegender Motor 25 trägt zu diesem Problem bei, da dem Innenraum die durch den Motor 25 generierte Wärme durch die Klimasteuerung 26 wieder entzogen werden muss.

Figs. 3a und 3b illustrieren mit einem schematischen Schnitt durch jeweils eine Ausführungsformen des Schüttlers einen weiteren Aspekt der Erfindung. Im Gegensatz zu Fig. 2 (Stand der Technik) ist das Tablar 31 hier über eine Hauptantriebswelle 33 von einem Motor 36 antreibbar, welcher ausserhalb des Gehäuses 34 angebracht ist. Die Hauptantriebswelle 33 verläuft orthogonal zum Tablar 31 und zum grösseren Teil innerhalb des Gehäuses 34, während ein kleinerer Teil der Hauptantriebswelle 33 ausserhalb des Gehäuses verläuft. Die Anbringung des Motors 36 unterhalb des Gehäuses 34 ist im Hinblick auf einen tiefen Schwerpunkt der Vorrichtung vorteilhaft.

Fig. 3b zeigt einen Schnitt durch eine Ausführungsform der Vorrichtung, bei welcher der Träger 32 über ein Lager 36a an einem Trägerelement 35, das am Gehäuse 34 befestigt ist, gelagert ist. Dadurch wird wie in Fig. 1c eine mechanische Entkopplung der Lagerung/Halterung des Trägers vom Antrieb, insbesondere von der Hauptantriebswelle 33, erreicht.

Ein ausserhalb des Gehäuses angebrachter Motor, insbesondere wie in Fig. 3a, hat allgemein den Vorteil, dass die vom Motor erzeugte Wärme nicht in den Innenraum des Gehäuses eingebracht wird und diesen damit nicht erwärmt. Dadurch ist weniger Kühlleistung erforderlich, um den Innenraum auf einer konstanten Temperatur zu halten. So kondensiert im Innenraum, z.B. lokal an der Klimasteuerung bzw. dem Kühler, auch weniger Feuchtigkeit aus, welche für die Proben schädlich sein könnte. Dies erleichtert das Schaffen kontrollierter Umgebungsbedingungen im Innenraum, insbesondere einer konstanten Temperatur und einer hohen Feuchtigkeit, z.B. zwischen 80% und 100% relativer Feuchte.

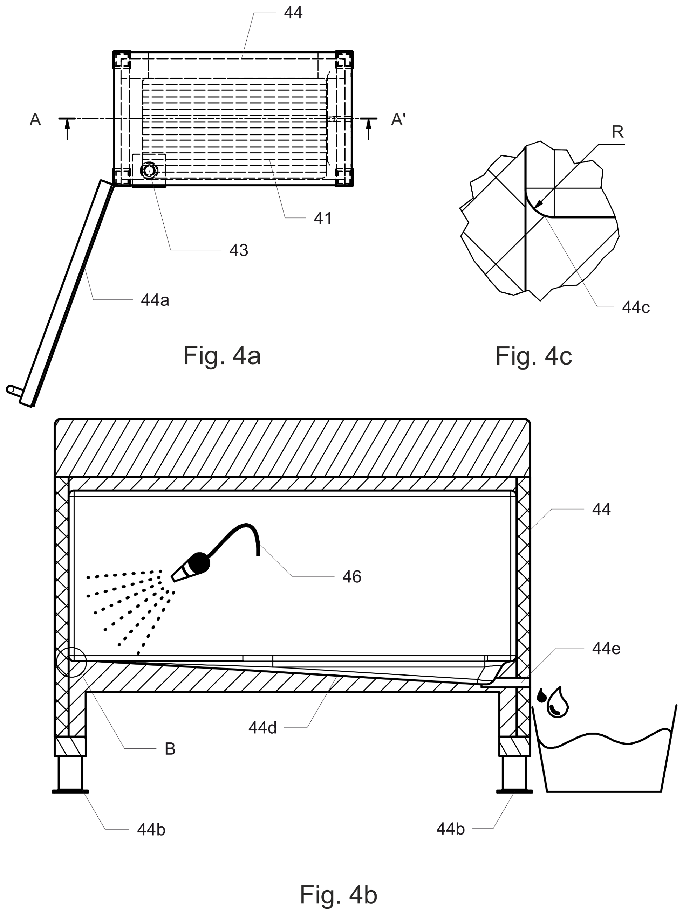

Figs. 4a bis 4c zeigen einen weiteren Aspekt der Erfindung, der die Reinigbarkeit verbessert, anhand einer Draufsicht auf die Vorrichtung (Fig. 4a), einem Schnitt durch die Vorrichtung (Fig. 4b, Tablar und Hauptantriebswelle nicht dargestellt) und einer Detailansicht eines Eckbereichs des Innenraums (Fig. 4c).

In der Draufsicht der Fig. 4a ist ein Gehäuse 44 umfassend eine Tür 44a im offenen Zustand gezeigt. Zudem sind das Tablar 41 sowie der Hauptantriebswelle 43 gestrichelt angedeutet.

Fig. 4b illustriert nun den Schnitt vertikalen Schnitt entlang der Linie AA' aus Fig. 4a. Das Gehäuse 44 umfasst Füsse 44b eingerichtet zum Tragen des Gehäuses 44. Die Füsse 44b definieren eine Auflagefläche, insbesondere als Ebene durch die Füsse 44b. Mit den Füssen 44b bzw. der Auflagefläche kann die Vorrichtung auf einem Untergrund, z.B. einem Tisch, abgestellt werden.

Der Innenraum des Gehäuses 44 umfasst zum einen abgerundete Ecken und Kanten 44c, siehe den Eckbereich B in Fig. 4b sowie dessen Detailansicht in Fig. 4c. Dabei ist es vorteilhaft, wenn zumindest eine Mehrzahl der Ecken und Kanten 44c des Innenraums abgerundet ist. "Abgerundet" soll insbesondere heissen, dass ein Rundungsradius R der Ecken und Kanten 44c mindestens 1 mm beträgt. Vorteilhafterweise beträgt der Rundungsradius R mindestens 10 mm, z.B. 15 mm. Dies verhindert, dass sich in den Ecken und Kanten Probenmaterial oder Schmutz ansammelt, der schwer zu reinigen wäre. So kann der Innenraum beispielsweise durch Ausspritzen, z.B. mit der Spritzdüse 46, ausreichend gereinigt werden.

Zum anderen kann die Unterseite 44d des Innenraums zusätzlich geneigt sein, also schräg zur Auflagefläche des Gehäuses verlaufen. Dadurch sammelt sich Flüssigkeit im Innenraum an der tiefsten, also Auflageflächen-nächsten, Stelle der Unterseite 44d. Dort befindet sich eine Auslassöffnung 44e durch das Gehäuse 44 hindurch, durch welche die Flüssigkeit abfliessen kann. Auch dies verbessert die Reinigbarkeit der Vorrichtung, z.B. durch die Möglichkeit des einfachen Ausspritzen des Innenraums.

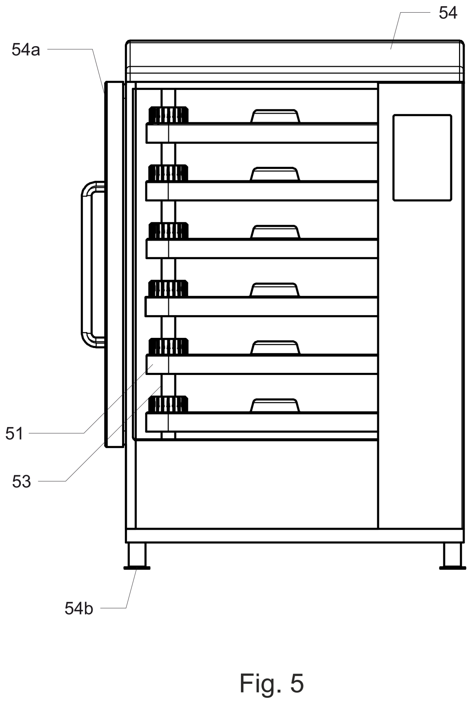

Fig. 5 illustriert einen Schüttler mit mehreren, insbesondere sechs, Tablaren 51, die in einem Gehäuse 54 mit Tür 54a von einer einzigen Hauptantriebswelle 53 antreibbar sind. So sind alle Tablare 51 von einem Motor (nicht gezeigt in Fig. 5) antreibbar, der wiederum ausserhalb des möglicherweise klimatisierten Innenraums des Gehäuses 54 angebracht ist, wie oben beschrieben. Vorteilhafterweise verläuft die Hauptantriebswelle 53 für eine optimale Schwenkbarkeit der Tablare 51 wiederum im Randbereich, insbesondere im Eckbereich, durch die Tablare 51 hindurch.

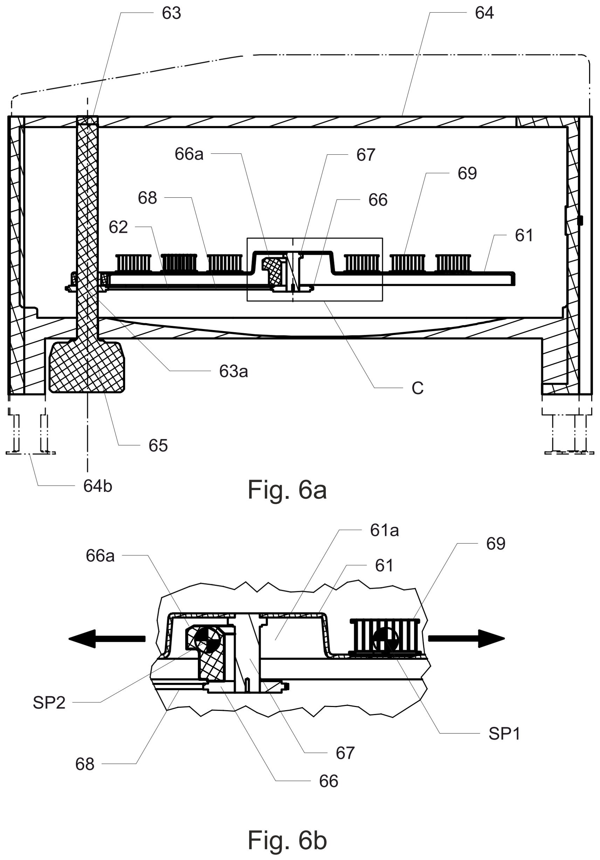

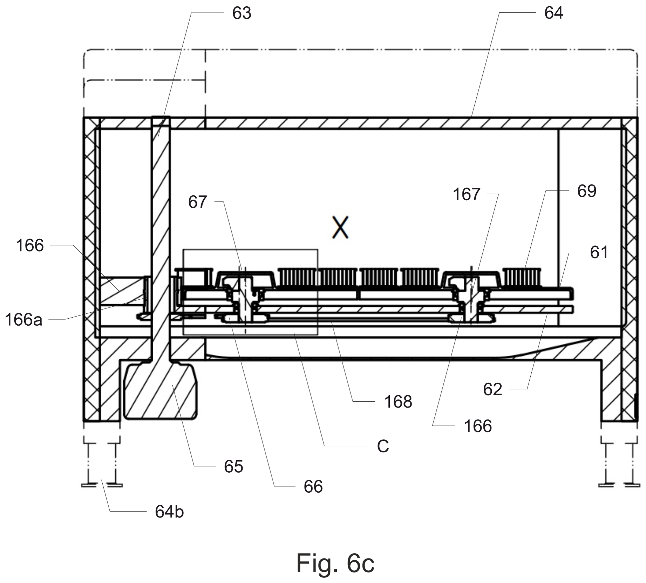

Figs. 6a und 6b fokussieren auf den mechanischen Aspekt, wie ein Tablar 61 über einen Träger 62 mit einem Lager 63a an der Hauptantriebswelle 63 schwenkbar befestigt ist (Fig. 6a), sowie Details zum Antrieb des Tablars 61 über ein Antriebselement 66 (Fig. 6b). Fig. 6c zeigt eine alternative Lösung der Lagerung des Tablars 61 über einen Träger 62 mit einem Lager 166a, insbesondere einem Gleitlager, an einem Trägerelement 166, das Teil des Gehäuses 64 oder an diesem befestigt ist. Prinzipiell sind die beschriebenen Mechanismen auch auf mehrere Tablare anwendbar, z.B. auf den Schüttler nach Fig. 5.

Das Tablar 61 ist dazu eingerichtet, mit einer oder mehreren Proben 69, z.B. in Mikrotiterplatten, beladen zu werden, die geschüttelt werden sollen. Hierfür weist das Tablar 61 bevorzugt Befestigungselemente, z.B. für einen Gefässständer auf, um die Proben 69 bzw. Gefässe, insbesondere Mikrotiterplatten, während des Schüttelvorgangs relativ zum Tablar 61 ortsfest zu halten.

Das Tablar 61 ist über eine fest verbundene Tablarwelle 67 drehbar an dem Antriebselement 66 gelagert. Das Antriebselement 66 ist wiederum an dem Träger 62 drehbar befestigt, welcher an der Hauptantriebswelle 63 über das Lager 63a schwenkbar gelagert ist. Die Lagerung der Tablarwelle 67 in oder an dem Antriebselement 66 ist exzentrisch, die Drehachse der Tablarwelle 67 fällt also nicht mit der Drehachse des Antriebselements 66 zusammen. Durch diese Exzentrizität der Tablarwelle 67 entsteht bei Rotation des Antriebselements 66 eine Kreisbewegung, auf der das Tablar 61 umläuft, und somit das gewünschte Schütteln des Tablars 61 samt Proben 69.

Optional wird das Tablar 61 im eingeschwenkten Zustand von einem Gehäuse 64 umschlossen, welches wie oben beschrieben als Verspritzschutz und/oder zur Klimatisierung der Proben dient. Die Hauptantriebswelle 63 verläuft, insbesondere vertikal, also in Richtung der Schwerkraft, durch das Gehäuse 64 hindurch und ist an diesem frei drehbar gelagert. Weiterhin ist ein Motor 65 zum Antreiben der Hauptantriebswelle 63, bevorzugt aussen, am Gehäuse 64 befestigt.

Ausserdem kann das Gehäuse 64 (wie bereits in Bezug auf Fig. 1a beschrieben) Füsse 64b umfassen, die dazu eingerichtet sind, das Gewicht der Vorrichtung zu tragen. Im Allgemeinen können die Füsse auch für eine Befestigung an einer Unterlage, z.B. an einem Labortisch, eingerichtet sein.

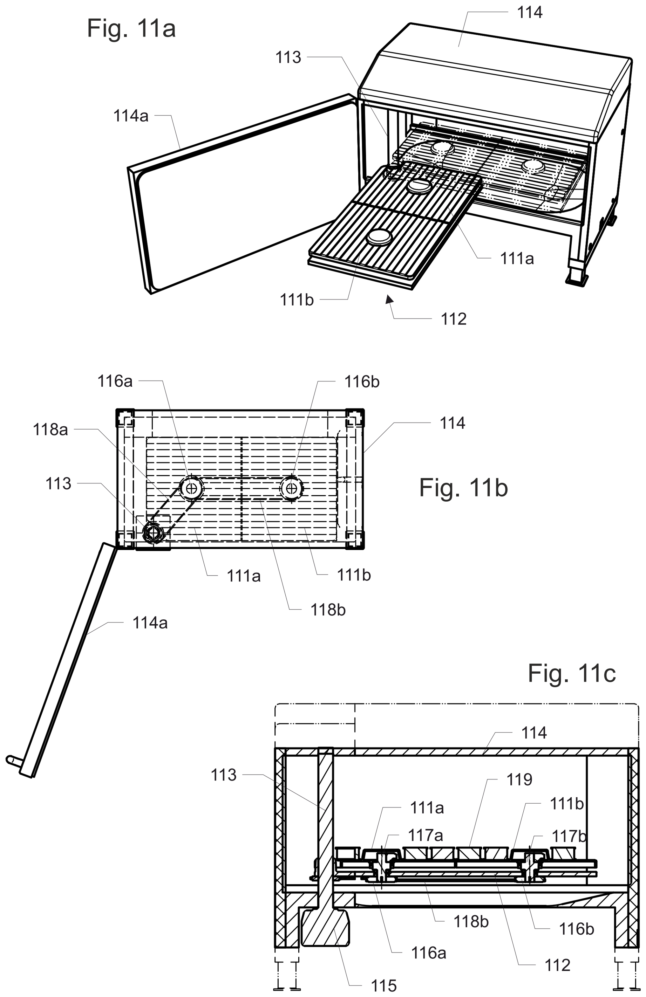

Fig. 6b ist eine vergrösserte Ansicht von Ausschnitt C in Figs. 6a und 6c. Das Tablar 61, das mit Proben 69, z.B. in einer Mikrotiterplatte, bestückt werden kann, ist über die Tablarwelle 67 drehbar an dem Antriebselement 66 gelagert. Das Antriebselement 66 umfasst bevorzugt eine Riemenscheibe, die über einen Riemen 68 von der Hauptantriebswelle angetrieben wird. Dazu ist an der Hauptantriebswelle eine zweite Riemenscheibe angebracht und der Riemen 68 ist über die beiden Riemenscheiben gespannt. Vorteilhafterweise ist das Tablar 61 auch an der zweiten Riemenscheibe in gleicher Weise exzentrisch gelagert wie an der ersten Riemenscheibe am Antriebselement 66. Dies stellt eine Verdrehsicherung für das Tablar 61 dar, da seine Bewegungsfreiheit damit auf eine kreisförmige Translation eingeschränkt wird. Ausserdem kann das Tablar zur Verdrehsicherung, wie oben beschrieben, zwei Tablarteile umfassen, die miteinander z.B. durch eine Linearführung verbunden sind. Eine solche Ausführungsform ist in Figs. 11a, 11b und 11c gezeigt, siehe unten.

Fig. 6c zeigt - unabhängig von der unterschiedlichen Lagerung des Trägers zu Fig. 6a - auch eine vorteilhafte Ausführung der Lagerung des Tablars 61 am Träger 62. Zusätzlich zu dem Antriebselement 66 und der Tablarwelle 67 umfasst die Vorrichtung ein zweites Antriebselement 166 und eine zweite Tablarwelle 167. Das Tablar 61 ist also sowohl mit der Tablarwelle 67 als auch mit der zweiten Tablarwelle 167 verbunden, welche exzentrisch - und zwar mit der gleichen Exzentrizität - an den Antriebselementen 66 bzw. 166 gelagert sind. Damit die beiden Antriebselemente 66 und 166 synchron rotieren sind sie mechanisch, z.B. über einen Zahnriemen 168, miteinander gekoppelt. Durch diese Lagerung des Tablars 61 am Träger 62 wird eine unerwünschte Rotation des Tablars 61 während der angestrebten Orbitalbewegung beim Schütteln verhindert.

Im Allgemeinen ist es vorteilhaft, Unwuchten, die an rotierenden Bauteilen der Vorrichtung auftreten, auszugleichen. Dies betrifft neben der Hauptantriebswelle, vgl. oben den Abschnitt "Mehrere Tablare", vor allem auch das Antriebselement 66. Insbesondere bei hohen Schüttelfrequenzen und daher Drehzahlen, z.B. von über 1000 rpm, über 1500 rpm, über 2000 rpm oder über 2500 rpm, z.B. bei 3 mm Durchmesser der Kreisbewegung, wie sie die Vorrichtung erreichen kann, führt eine Unwucht sonst zu Vibrationen, zu erhöhtem Verschleiss der Lager und Lagerungen sowie zu übermässiger Lärmentwicklung.

Fig. 6b zeigt eine Anordnung eines Gegengewichts 66a an dem Antriebselement 66, welche besonders einfach und wirkungsvoll eine Unwucht ausgleicht, die durch die exzentrische Lagerung des Tablars 61 (mit Proben 69) an dem Antriebselement 66 hervorgerufen wird. Dabei ist es vorteilhaft, dass sich der Schwerpunkt SP2 des Gegengewichts 66a in der gleichen, orthogonal zur Drehachse des Antriebselements 66 verlaufenden Ebene befindet wie der Schwerpunkt SP1 des Tablars 61 samt der vorgesehenen Beladung mit Proben 69. Dies kann gemäss Fig. 6b so gelöst werden, dass das Tablar 61 eine Ausstülpung 61a nach oben umfasst, unter welcher sich zumindest ein Teil des Gegengewichts 66a befindet. Die Drehmomente, die bei Rotation des Antriebselements 66 um die Drehachse durch SP1 und SP2 ausgeübt werden, sollen sich im Allgemeinen gerade aufheben. Mit der gezeigten Anordnung lassen sich sowohl eine statische als auch eine dynamische Unwucht ausgleichen. Dies ermöglicht es, mit einem platzsparenden Aufbau hohe Schüttelfrequenzen von über 1000 rpm, insbesondere über 1500 rpm, über 2000 rpm oder über 2500 rpm, bei z.B. 3 mm Durchmesser der Kreisbewegung zu erreichen.

Auch bei der Ausführungsform von Fig. 6c sind aus den gleichen Gründen vorteilhafterweise an den Antriebselementen 66 und 166 Gegengewichte angeordnet. Diese Gegengewichte sind wiederum (analog zur obigen Beschreibung) so angepasst, dass sie sowohl eine statische als auch eine dynamische Unwucht bei der Orbitalbewegung des Tablars 61 ausgleichen.

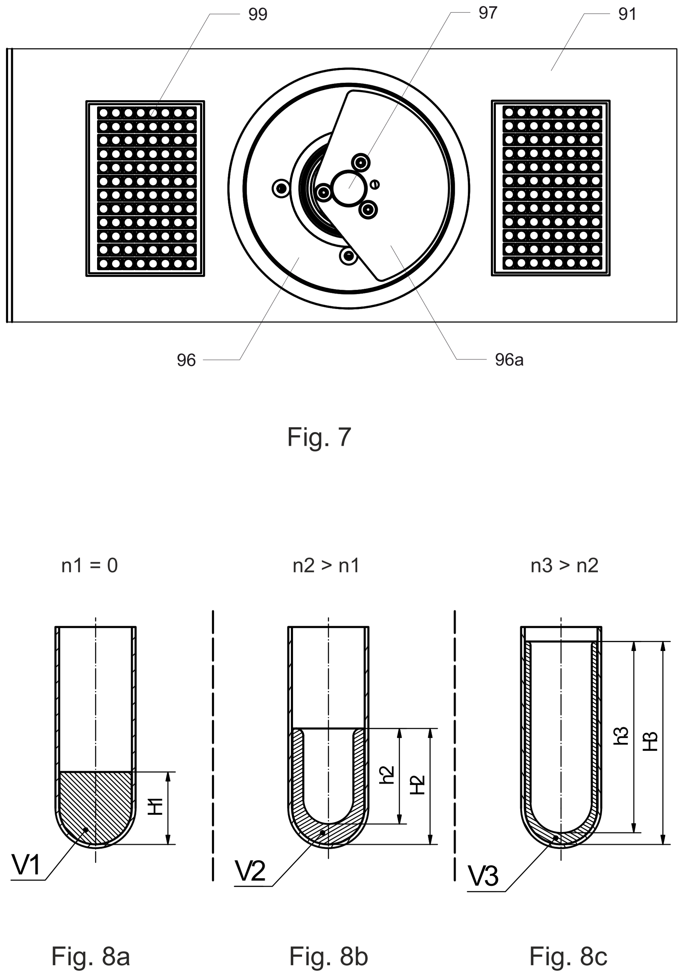

Fig. 7 zeigt eine Draufsicht von oben auf bzw. einen horizontalen Schnitt durch ein Tablar 91 mit Gegengewicht 96a. Auf dem Tablar 91 sind z.B. mittels Gefässhaltern zwei Mikrotiterplatten mit einer Vielzahl von Proben 99 angebracht. Wie oben im Zusammenhang mit Fig. 6b beschrieben, ist das Gegengewicht 96a an dem Antriebselement 96 angebracht, sodass besonders einfach und wirkungsvoll eine Unwucht ausgeglichen wird, die durch die exzentrische Lagerung des Tablars 91 (mit Proben 99) an dem Antriebselement 96 hervorgerufen wird. Wie gezeigt kann das Gegengewicht 96a z.B. mit Schrauben an dem Antriebselement 96 befestigt sein. Wiederum befindet sich der Schwerpunkt des Gegengewichts 96a in der gleichen, orthogonal zur Drehachse des Antriebselements 96 verlaufenden Ebene wie der Schwerpunkt des Tablars 91 samt der vorgesehenen Beladung mit Proben 99.

Vorteilhafterweise umfasst das Gegengewicht 96a eine Öffnung, durch welche die Tablarwelle 97 hindurch verläuft. Ausserdem kann das Gegengewicht 96a in der Draufsicht von oben vorteilhafterweise ähnlich einem Kreissektor geformt sein. Beide Ausgestaltungen ermöglichen ein möglichst grosses Volumen und damit eine möglichst grosse Masse des Gegengewichts 96a, wobei das Gegengewicht 96a dennoch mit dem Antriebselement 96 in der Ausstülpung des Tablars 91 umlaufen kann. Dadurch wird der für die Proben 99 auf dem Tablar 91 verfügbare Platz maximiert.

Figs. 8a, 8b und 8c illustrieren den Effekt verschiedener Schüttelfrequenzen n1, n2 bzw. n3 auf eine flüssige Probe, die sich in einem Gefäss, z.B. einem Reagenzglas oder einer Mikrotiterplatte, befindet. Das Probenvolumen V ist in jeder der Abbildungen gleich: V1 = V2 = V3.

In Fig. 8a ist die Probe in Ruhe, also Schüttelfrequenz n1 = 0. Die Probenflüssigkeit weist eine annähernd ebene und waagrechte Oberfläche auf. Die Probenflüssigkeit füllt das Gefäss dabei bis zu einer Höhe H1. Diese Höhe kann als Mass für die Diffusionsstrecke d1 genommen werden, welche Sauerstoff aus dem umgebenden Gas in die Probe hinein zurücklegen muss: d1 = H1.

In Fig. 8b wird die Probe mit einer Schüttelfrequenz n2 > 0 geschüttelt, z.B. mit n2 = 1000 rpm. Dabei wird die Flüssigkeit am Gefässrand nach oben gedrückt und es bildet sich ein Meniskus, also eine konkave Oberfläche der Probenflüssigkeit aus. Während die Oberfläche gegenüber der Situation in Fig. 8a zunimmt, nimmt die Diffusionsstrecke d2 = H2-h2 ab: d2 < d1. Beides führt dazu, dass Sauerstoff schneller in die Probe gelangt.

In Fig. 8c wird die Probe noch deutlich schneller geschüttelt, n3 > n2, z.B. mit n3 = 2000 rpm. Die Probenflüssigkeit wird weit am Gefässrand nach oben "gezogen". Die Oberfläche nimmt infolge des sich verstärkenden Meniskus weiter zu und die Diffusionsstrecke d3 = H3-h3 nimmt weiter ab: d3 < d2. Der Sauerstofftransport in die Probe ist also nochmals verbessert.

Damit ist einer der grossen Vorteile der beschriebenen Vorrichtung gezeigt, welche Schüttelfrequenzen von über 1000 rpm, insbesondere über 1500 rpm, über 2000 rpm oder über 2500 rpm, erreichen kann: Der Sauerstofftransport aus der Gasphase in die Flüssigphase, also in die Probe hinein, wird stark erhöht. Insbesondere lassen sich so Zellen mit einem ähnlichen schnellen Wachstum kultivieren und ähnlich viel Biomasse produzieren wie bei einer Kultivierung in einem Bioreaktor. Mit einer solchen Vorrichtung können also bereits erste Tests bei der Zellkultivierung unter ähnlichen Bedingungen durchgeführt werden wie später im ausgereiften Prozess, insbesondere im Bioreaktor.

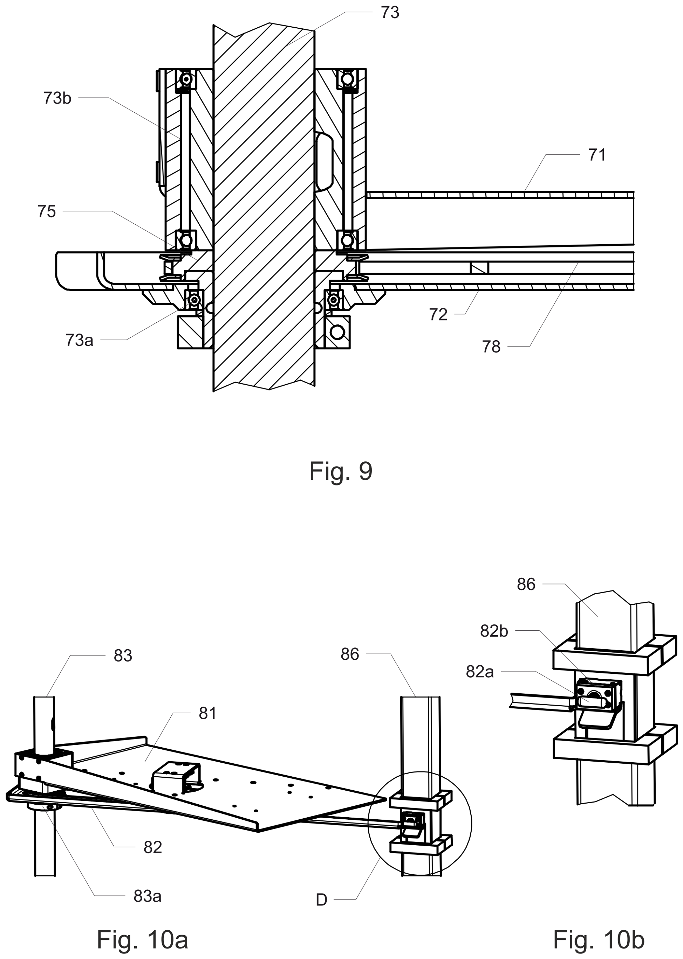

Fig. 9 zeigt, wie ein Tablar 71 über einen Träger 72 an einer Hauptantriebswelle 73 gelagert sein kann. Eine solche Lagerung ist z.B. kompatibel mit den Ausführungsformen der Figs. 1a/b, 3, 5 und 6a/b. Wie in Figs. 6a/b ist das Tablar 71 exzentrisch an einem Antriebselement (nicht gezeigt) mit erster Riemenscheibe gelagert. Das Antriebselement bzw. die erste Riemenscheibe ist an dem Träger 72 drehbar gelagert und eingerichtet, über den Riemen 78 angetrieben zu werden. Der Riemen 78 verläuft zusätzlich über die zweite Riemenscheibe 75, welche an der Hauptantriebswelle 73 befestigt ist und entsprechend vom Antrieb bzw. Motor über die Hauptantriebswelle 73 angetrieben wird.

Der Träger 72, welcher dazu eingerichtet ist, das Gewicht des Tablars 71 samt Beladung mit den Proben abzustützen, ist über ein Lager 73a, z.B. ein Kugellager, an der Hauptantriebswelle 73 gelagert. So kann der Träger 72 ortsfest bleiben, z.B. indem er über eine Verrastung wie in Figs. 10a/b arretiert wird, während die Hauptantriebswelle 73 rotiert. Bevorzugt befindet sich das Lager 73a unterhalb der zweiten Riemenscheibe 75.

Optional kann der Träger 72 samt Tablar 71 alternativ oder zusätzlich auch oberhalb der zweiten Riemenscheibe 75 über ein Lager 73b an der Hauptantriebswelle 73 gelagert sein. Im Allgemeinen ist darauf zu achten, dass das Tablar für seine kreisförmige Translation, welche durch die exzentrische Lagerung verursacht wird, genügend Spielraum hat. Insbesondere muss eine Öffnung in dem Tablar 71, durch welche die Hauptantriebswelle 73 in einer bevorzugten Ausführungsform hindurchtritt, mindestens um die Exzentrizität der Lagerung grösser sein als der Durchmesser der Hauptantriebswelle 73 bzw. als das zweite Lager 73b, wenn vorhanden.

Mit einem Lager 73a bzw. 73b lassen sich auch mehrere Träger übereinander an einer Hauptantriebswelle 73 schwenkbar anbringen und gleichzeitig antreiben.

Figs. 10a und 10b illustrieren eine Möglichkeit, ein Tablar 81, das (wie z.B. im Zusammenhang mit Figs. 6a/b und 9 beschrieben) über ein Antriebselement (nicht sichtbar) an einem Träger 82 befestigt ist, für den Schüttelvorgang im Gehäuse bzw. an einer Stützstruktur 86 im Gehäuse zu arretieren, sodass es vorübergehend nicht um die Hauptantriebsachse schwenkbar ist. Die Stützstruktur 86 kann dabei Teil des Gehäuses oder ein separates Bauteil sein, das am Gehäuse befestigt ist. Für die Schwenkbarkeit des Tablars 81 ist der Träger 82 wiederum über ein Lager 83a an der Hauptantriebswelle 83 schwenkbar gelagert, siehe z.B. Fig. 9.

Fig. 10b zeigt den Ausschnitt D von Fig. 10a vergrössert. Dabei umfasst ein erstes Arretierelement 82a als Verrastung an oder nahe seinem Hauptantriebswellen-fernen Ende. Als Gegenstück zum ersten Arretierelement 82a ist an der Stützstruktur 86 ein zweites Arretierelement 82b angebracht. Das erste und zweite Arretierelement 82a und 82b sind insbesondere dazu eingerichtet, bei Kontakt eine lösbare Verbindung herzustellen. Dadurch kann der Träger 82 für den Schüttelvorgang mit der Stützstruktur 86 verbunden und insbesondere ein Schwenken des Trägers 82 um die Hauptantriebswelle 83 verhindert werden. Zusätzlich kann ein Teil des Gewichts von Träger 82 und Tablar 81 samt Proben von der Stützstruktur 86 getragen werden, was eine Belastung des Lagers 83a an der Hauptantriebsachse vermindert.

Im Allgemeinen umfasst die Verrastung z.B. mechanische oder magnetische Komponenten zum lösbaren Verbinden des Trägers 82 mit der Stützstruktur 86. Beispielsweise können die Arretierelemente 82a und 82b Magneten umfassen, welche dazu eingerichtet sind, durch ihre gegenseitige Anziehung den Träger 82 an der Stützstruktur 86 zu arretieren. Alternativ können die Arretierelemente 82a und 82b als Schnappverschluss oder als Klappverschluss ausgebildet sein, bei dem mechanisch eine lösbare Verbindung hergestellt wird.

Figs. 11a, 11b und 11c illustrieren eine Ausführungsform des Schüttlers mit geteiltem Tablar, wodurch eine besonders einfache und zuverlässige Verdrehsicherung für das Tablar erreicht werden kann. Fig. 11a ist eine perspektivische Ansicht analog zu Fig. 1a; Fig. 11b ist ein schematischer Schnitt durch den Schüttler in der Ebene der Antriebselemente analog zu Fig. 1b; Fig. 11c ist ein schematischer vertikaler Schnitt analog zu Fig. 6a. Die zu den früheren Ausführungsformen beschriebenen Merkmale sind hier analog anwendbar.

Der Schüttler umfasst ein Gehäuse 114 mit einer Tür 114a, die zum Öffnen und Schliessen einer Gehäusevorderseite eingerichtet ist. In Figs. 11a und 11b ist die Tür 114a in geöffnetem Zustand dargestellt. Weiterhin umfasst der Schüttler eine Hauptantriebswelle 113, die von einem Motor 115 antreibbar ist. An der Hauptantriebswelle 113 ist ein Träger 112 drehbar gelagert, der z.B. mittels einer Verrastung (wie oben beschrieben) am Gehäsue eingerastet werden kann. Am Träger 112 sind wiederum ein erstes Antriebselement 116a und ein zweites Antriebselement 116b drehbar gelagert. Das erste Antriebselement 116a ist über einen ersten Riemen 118a an die Hauptantriebsachse 113 gekoppelt und wird von dieser angetrieben. Das zweite Antriebselement 116b ist über einen zweiten Riemen 118b an das erste Antriebselement 116a gekoppelt und wird damit ebenfalls angetrieben. Wichtig ist, dass die beiden Antriebselemente 116a und 116b synchron laufen. Daher wird zumindest für den zweiten Riemen 118b vorteilhafterweise ein Zahnriemen eingesetzt.

An oder in dem ersten Antriebselement 116a bzw. dem zweiten Antriebselement 116b ist eine erste Tablarwelle 117a bzw. eine zweite Tablarwelle 117b exzentrisch gelagert. An der ersten Tablarwelle 117a bzw. an der zweiten Tablarwelle 117b ist wiederum ein erster Tablarteil 111a bzw. ein zweiter Tablarteil 111b, insbesondere verdrehsicher, befestigt. Auf den Tablarteilen 111a und 111b können, wie früher beschrieben, Proben 119 angebracht werden, z.B. in Reagenzgläsern oder Mikrotiterplatten.

Eine besonders einfache und zuverlässige Verdrehsicherung für die beiden Tablarteile 111a und 111b kann nun über eine flexible Verbindung der beiden Tablarteile erreicht werden (in Figs. 11a-c nicht gezeigt). Vorteilhafterweise umfasst diese Verbindung eine Linearführung zwischen dem ersten Tablarteil 111a und dem zweiten Tablarteil 111b. Die Linearführung kann z.B. am einen Tablarteil fix befestigt sein, während sie eine Verschiebung des anderen Tablarteils entlang der Führung zulässt. Durch eine solche flexible Verbindung werden schädliche Kräfte auf die Lager der Tablarwellen und Antriebselemente, z.B. infolge von Wärmeausdehnung insbesondere des Trägers 112, vermieden.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Vorrichtung zum Schütteln von Proben umfassend
- einen Träger (62),
- ein Antriebselement (66), das drehbar am Träger (62) gelagert ist,
- ein Tablar (61) eingerichtet zur Beladung mit den Proben (69),
- eine Tablarwelle (67), die mit dem Tablar (61) verbunden und exzentrisch an dem Antriebselement (66) gelagert ist,
- ein öffenbares Gehäuse (64), wobei sich das Tablar (61), die Tablarwelle (67), das Antriebselement (66) und der Träger (62) in einem Innenraum innerhalb des Gehäuses (64) befinden,
- eine durch einen Antrieb (65) antreibbare Hauptantriebswelle (63), die orthogonal zu dem Tablar (61) zumindest teilweise durch das Gehäuse (64) verläuft,
wobei das Antriebselement (66) über die Hauptantriebswelle (63) antreibbar ist,
wobei der Träger (62) samt Tablar (61), insbesondere mit einem Lager (63a, 166a), um die Hauptantriebswelle (63) schwenkbar gelagert ist,
wobei der Träger (62) samt Tablar (61) zumindest teilweise aus dem Gehäuse (64) im geöffneten Zustand heraus schwenkbar ist.

2. Vorrichtung nach Anspruch 1,
wobei der Träger (62) samt Tablar (61) zu mehr als 50% einer Fläche des Tablars (61) aus dem Gehäuse (64) im geöffneten Zustand heraus schwenkbar ist.

3. Vorrichtung nach einem der vorangehenden Ansprüche,
wobei der Träger (62) samt Tablar (61) um mindestens 45°, insbesondere mindestens 90°, um die Hauptantriebswelle (63) schwenkbar ist.

4. Vorrichtung nach einem der vorangehenden Ansprüche,
wobei das Tablar (11, 61) eine Öffnung (11a) umfasst, durch welche die Hauptantriebswelle (13, 63) hindurchtritt,
wobei sich die Öffnung (11a) in einem Randbereich des Tablars (11, 61) befindet, insbesondere weniger als 20% einer Länge und/oder Breite des Tablars (11, 61) von einem Rand des Tablars (11, 61) entfernt.

5. Vorrichtung nach einem der Ansprüche 1 bis 3,
wobei die Hauptantriebswelle (63) ausserhalb der Fläche des Tablars (61) verläuft.

6. Vorrichtung nach einem der vorangehenden Ansprüche,
wobei der Träger (62, 82) am Hauptantriebswellen-fernen Ende eine Verrastung (82a) zum lösbaren Befestigen des Trägers (62, 82) an dem Gehäuse (64) oder an einer Stützstruktur (86) im Gehäuse umfasst.

7. Vorrichtung nach einem der vorangehenden Ansprüche, weiterhin umfassend
- einen Riemen (68) eingerichtet zum Antreiben des Antriebselements (66) über die Hauptantriebswelle (63).

8. Vorrichtung nach einem der vorangehenden Ansprüche,
wobei die Tablarwelle (67) zentral an dem Tablar (61) befestigt ist.

9. Vorrichtung nach einem der vorangehenden Ansprüche,
wobei das Tablar (61) gegen Rotation gegenüber dem Träger (62) gesichert ist.

10. Vorrichtung nach Anspruch 9,
wobei das Tablar einen ersten Tablarteil (111a) und einen zweiten Tablarteil (111b) umfasst,
wobei der erste Tablarteil (111a) und der zweite Tablarteil (111b) über eine erste Tablarwelle (117a) bzw. eine zweite Tablarwelle (117b), die mit dem ersten bzw. dem zweiten Tablarteil verbunden sind, exzentrisch an einem ersten Antriebselement (116a) bzw. einem zweiten Antriebselement (116b) gelagert sind,
wobei das erste Antriebselement (116a) und das zweite Antriebselement (116b) drehbar am Träger (112) gelagert und über die Hauptantriebswelle (113) antreibbar sind,
wobei der erste und der zweite Tablarteil durch eine flexible Verbindung, insbesondere durch eine Linearführung, miteinander verbunden sind.

11. Vorrichtung nach einem der vorangehenden Ansprüche, weiterhin umfassend
- mindestens ein weiteres Tablar (51, 61), insbesondere mindestens fünf weitere Tablare, samt weiterer Tablarwelle, weiterem Antriebselement und weiterem Träger im Innenraum des Gehäuses,
wobei das mindestens eine weitere Antriebselement ebenfalls über die Hauptantriebswelle (53, 63) antreibbar ist.

12. Vorrichtung nach Anspruch 11,
wobei das mindestens eine weitere Tablar (51, 61) unabhängig von dem anderen Tablar bzw. den anderen Tablaren um die Hauptantriebswelle (53, 63) schwenkbar gelagert ist.

13. Vorrichtung nach einem der Ansprüche 11 bis 12,
wobei eine Winkelposition einer Lagerung der weiteren Tablarwelle an dem weiteren Antriebselement abweicht von einer Winkelposition der Lagerung der Tablarwelle an dem Antriebselement,
insbesondere wobei sich die Winkelposition der Lagerungen der verschiedenen Tablarwellen zueinander um 360°/N unterscheiden, wobei N die Anzahl der Tablare in der Vorrichtung ist.

14. Vorrichtung nach einem der vorangehenden Ansprüche,
weiterhin umfassend ein Klimasteuerelement eingerichtet zum Steuern von Temperatur und/oder Feuchtigkeit im Innenraum des Gehäuses.

15. Vorrichtung nach einem der vorangehenden Ansprüche, wobei der Antrieb (65) einen Motor umfasst, der ausserhalb des Gehäuses (64), insbesondere an einer Unterseite des Gehäuses (64), angebracht ist,
insbesondere wobei die Hauptantriebswelle (63) durch eine Öffnung in der Unterseite des Gehäuses (64) hindurchgeführt ist, und/oder
insbesondere wobei der Innenraum des Gehäuses (64) thermisch von dem Motor entkoppelt ist.

16. Vorrichtung nach einem der vorangehenden Ansprüche, wobei eine Exzentrizität der Lagerung der Tablarwelle (67) an dem Antriebselement (66) zwischen 0.5 und 50 mm beträgt, insbesondere zwischen 1 und 3 mm.

17. Vorrichtung nach einem der vorangehenden Ansprüche, wobei eine Schüttelfrequenz des Tablars (61) infolge des Antriebs (65) mindestens 1000 rpm, insbesondere mindestens 1500 rpm, mindestens 2000 rpm oder mindestens 2500 rpm, beträgt.

18. Vorrichtung nach einem der vorangehenden Ansprüche,
wobei das Tablar (61) eine rechteckige Form aufweist, insbesondere wobei das Tablar (61) eine Länge zwischen 50 und 100 cm aufweist, und/oder
insbesondere wobei das Tablar (61) eine Breite zwischen 30 und 70 cm aufweist.

19. Vorrichtung nach einem der vorangehenden Ansprüche,
wobei ein dem Innenraum zugewandter Teil des Gehäuses (64) zumindest zu 50% mit rostfreiem Stahl bedeckt ist.

20. Vorrichtung nach einem der vorangehenden Ansprüche,
wobei der dem Innenraum zugewandte Teil des Gehäuses (44, 64) abgerundete Ecken und Kanten (44c) umfasst,
insbesondere wobei ein Radius (R) der Ecken und Kanten (44c) mindestens 10 mm beträgt.

21. Vorrichtung nach einem der vorangehenden Ansprüche,
wobei zumindest ein Teil, insbesondere mindestens 70%, einer Unterseite (44d) des Innenraums gegenüber einer Auflagefläche des Gehäuses (44, 64) schräg verläuft,
insbesondere wobei ein Winkel zwischen der Unterseite (44d) des Innenraums und der Auflagefläche des Gehäuses (44, 64) zwischen 1° und 30°, insbesondere zwischen 5° und 15°, beträgt.

22. Vorrichtung nach Anspruch 21,
wobei das Gehäuse (44, 64) im Auflagefläche-nächsten Bereich der Unterseite (44d) des Innenraums eine Auslassöffnung (44e), insbesondere durch das Gehäuse (44, 64) hindurch, umfasst, die insbesondere verschliessbar ist.

23. Vorrichtung nach einem der vorangehenden Ansprüche, weiterhin umfassend
- ein am Gehäuse (64) befestigtes Trägerelement (166),
wobei der Träger (62) samt Tablar (61) über das Lager (166a), insbesondere über ein Gleitlager, schwenkbar an dem Trägerelement (166) gelagert ist,
insbesondere wobei eine Schwenkachse des Trägers (62) mit der Hauptantriebswelle (63) zusammenfällt.
